# EUROPEAN PATENT APPLICATION

(11) **EP 4 588 420 A1**
(43) Date of publication of application: **23.07.2025**
(21) Application number: 25152940.0
(22) Date of filing: 20.01.2025
(51) Int. Cl.: A61B 1/00

(54) **OPERATING INTERFACE, SURGICAL INSTRUMENT AND METHOD FOR MANUFACTURING AN OPERATING INTERFACE**

(30) Priority: 22.01.2024 DE 102024101691
(71) Applicant: Karl Storz SE & Co. KG, 78532 Tuttlingen (DE)
(72) Inventor: JUHANSON, Timo, 78532 Tuttlingen (DE)

(57) **Abstract**

The present invention provides an operating interface (1) for a surgical instrument (100), in particular for an endoscope, comprising: a housing (2) having a first part (2a) and a second part (2b), which is mountable to the first part (2a); and an accessory port (3) for transmitting fluids between an inside and an outside of the housing (2), wherein the accessory port (3) is integrated in the housing (2) and pivotable about a rotation axis (X) in relation to the housing (2). Further the present invention provides a surgical instrument (100) comprising such an operating interface (1) as well as a method for manufacturing an operating interface for a surgical instrument.

## Description

### TECHNICAL FIELD

The present invention pertains to an operating interface for a surgical instrument, a surgical instrument with such an operating interface and a method for manufacturing an operating interface for a surgical instrument.

### BACKGROUND

Surgical instruments exist in a wide variety of forms, for example in the form of endoscopes. In general, an endoscope comprises an operating interface at a proximal end (in use facing towards the user/operator/surgeon) and an insertion tube extending from the operating interface towards a distal end (in use facing towards the patient). The operating interface can be configured as a handle, which is adapted to be held by an operator and inter alia comprises externally protruding operating members connected to internal control means allowing the operator to control the movement of a bending section at the distal end of the insertion tube, while advancing the distal end of the insertion tube to a desired location e.g. within a body cavity of a patient/person. Advancing the distal end of the endoscope to a desired location, may involve many bends and turns where the operator turns the handle about the general longitudinal axis of the insertion tube to bring the angular orientation of insertion tube, and hence the bending section into a position from which the bending section may be bent to point towards a desired direction of view and/or of further advancement of the insertion tube. These many bends and turns require complicated and often awkward movements of not only the hand or wrist of the operator, but also elbow, arm shoulder, and sometimes even the torso and legs of the operator. While doing so the only real references the operator has is a monitor with up and down directions defined by the camera at the tip of distal end of the insertion. These directions, in turn, are linked to the handle to which the insertion tube is rigidly connected. During such movements the operator therefore should not be disturbed by accessories that are connected to the handle and also other entities arranged around the operator.

For example, surgical tube shaft instruments (e.g. endoscopes) are designed as reusable tube shaft instruments. Depending on the design of the instrument, the shaft, the insulating shaft and the operating interface in particular can be completely separated from each other for cleaning the instrument. After cleaning, the surgical instrument is reassembled and sterilized.

### DISCLOSURE OF THE INVENTION

It is an object of the present invention to provide an operating interface for easy and flexible use, in particular when equipped with accessories.

According to the invention, this problem is solved by an operating interface for a surgical instrument with the features of claim 1, by a surgical instrument with the features of claim 11 and/or by a method for manufacturing an operating interface for a surgical instrument with the features of claim 13.

According to a first aspect of the invention, an operating interface for a surgical instrument, in particular for an endoscope, is provided. The operating interface comprises a housing having a first part and a second part, which is mountable to the first part. Furthermore, the operating interface comprises an accessory port for transmitting fluids between an inside and an outside of the housing. The accessory port is integrated in the housing and pivotable about a rotation axis in relation to the housing.

According to a second aspect of the invention, a surgical instrument, in particular endoscope, is provided. The surgical instrument comprises an operating interface according to the first aspect of the invention and a shaft, which encloses a working channel. The shaft is coupled to the operating interface.

According to a third aspect of the invention, a method for manufacturing an operating interface for a surgical instrument, in particular for manufacturing an operating interface according to the first aspect of the invention, is provided. The method comprises the steps of:
Providing a first part of a housing.
Inserting an accessory port for transmitting fluids be-tween an inside and an outside of the housing into the first part of the housing.
Mounting a second part of the housing to the first part of the housing, wherein the accessory port is integrated in the housing and pivotable about a rotation axis in relation to the housing.

A fundamental concept of the invention is to provide a rotatable or rotating accessory port for use in a surgical instrument, in particular for use in a single use flexible endoscope. The present invention, for example, discloses a single use flexible endoscope having the accessory port in conjunction with the working channel for urology use. The accessory port can be fluidly connected to the working channel, which is arranged inside the housing of the operating interface.

A lower part of the accessory port that the working channel attaches to can be rigidly mounted in the surgical instrument, in particular in the operating interface. An upper part can rotate. The upper part of the accessory port is outside of the housing, in particular outside of the endoscope handle. Rotating connections can be configured water tight, for instance.

A particular advantage in the solution according to an aspect of the invention is that the need to use an external accessory having a rotating port when connecting to the surgical instrument can be omitted. A movement of the operating interface is not limited by the accessory port connection hose, in particular in comparison to the traditional design, where the irrigation port is fixed in relation to the operating interface.

Rotatable accessories do not need to be attached to the surgical instrument, in particular to the operating interface. Furthermore, rotatable accessories do not need to be sold separately. With the present invention, the surgical instrument, in particular endoscope, can be used more ergonomic.

The operating interface and/or the surgical instrument can be made of a plastic material. For example, the surgical instrument can be completely made of a plastic material, in particular when the surgical instrument is intended to use only once.

The working channel can be inserted into a tubular shaft of the surgical instrument. The working channel is mounted or guided at least at an end, in particular an proximal end, of a rod in the surgical instrument. In particular, the rod is mounted or guided in the tubular shaft in such a way that the working channel cannot move or rotate axially relative to the tube shaft.

A tool, which can be designed in particular in the form of an interchangeable accessory, can be fluidly coupled to the accessory port.

If the surgical instrument is to be operated by a user such as a surgeon, the surgical instrument comprises a handle as a grip for the user. The operating interface then transmits a force or torque, which is applied by the user to the handle via a suitable actuation (e.g. movable handle leg), to a force transmission element for example.

If the surgical instrument is to be connected to a robot, the surgical instrument comprises a corresponding connection interface for the robot, which can apply an axial force or torque to the force transmission element via the operating interface.

Advantageous embodiments and further developments emerge from the description with reference to the figures.

According to some embodiments of the invention, the accessory port is integrated in the housing by a form fit. For example, the accessory port can be enclosed at least section wise, in particular circumferentially, by the housing. The form fit can be provided by the first part and the second part of the housing.

According to some further embodiments of the invention, the accessory port comprises an end, which is arranged inside the housing and received in a working channel, and a projection element, which projects from a shell surface of the accessory port and is configured to provide the form fit in interaction with the housing.

According to some further embodiments of the invention, the accessory port comprises at least one luer connector, which is arranged outside the housing, wherein the accessory port is pivotable about the rotation axis such that an orientation of the at least one luer connector changes in relation to an orientation of the housing. Hence, the at least one luer connector can be rotated to both sides. Another benefit is that a connection to the at least one luer connector, which could be configured as an irrigation port, can be done on either side of the surgical instrument by an assistant when a doctor is holding the surgical instrument, for example.

According to some further embodiments of the invention, the accessory port comprises a first luer connector having a first longitudinal axis and a second luer connector having a second longitudinal axis, wherein the first longitudinal ax-is substantially corresponds to the rotation axis of the accessory port. A traditional accessory port of a flexible endoscope is fixed to the endoscope and uses two luer connectors as a T-piece, both connectors having a 90 degrees offset from each other. The first luer connector or the straight connection, respectively, can be used for accessories and tools used through the surgical instrument. The second luer connector or the 90 degrees offset luer connector, respectively, can be used for irrigation.

According to some further embodiments of the invention, the second longitudinal axis is substantially perpendicular in relation to the first longitudinal axis. For example, the accessory port can have a T-shape. Alternatively or additionally, an angle between the first longitudinal axis and the second longitudinal axis can be in a range from 65 ° to 115 °.

According to some further embodiments of the invention, an intersection of the first longitudinal axis and the second longitudinal axis is arranged inside the housing such that the second luer connector protrudes through a recess of the housing. That means the intersection corresponds to a bifurcation point. In particular, the first luer connector and the second luer connector protrude through different re-cesses of the housing.

According to some further embodiments of the invention, an intersection of the first longitudinal axis and the second longitudinal axis is arranged outside the housing.

According to some further embodiments of the invention, the accessory port comprises means, which are configured to support an orientation of the accessory port at a predefined rotation angle. The predefined rotation angle could be a central orientation of the accessory port and/or an orienta-tion with a rotation angle of about 90 ° in relation to the central orientation. For example, the means are configured such that the accessory port can click in to place in the predefined rotation angle.

According to some further embodiments of the invention, the operating interface is configured as an endoscope handle for manual operation by a user. Hence, the second luer con-nector can be pivoted on either side of the endoscope han-dle, for example to be used by an assistant for irrigation. Therefore, the user can operate the endoscope handle with his left or his right hand and pivot the accessory port such that the user is not disturbed by an accessory connected to the second luer connector. The user can be a doctor, in particular a surgeon.

According to some further embodiments of the invention, the shaft and the working channel are configured as a round tube, and wherein the shaft is arranged concentric in the operating interface.

The housing can consist of the first part and the second part. Furthermore, the first part and the second part each form a half of the housing. The housing can be divided lengthwise by the first part and the second part.

For example, the surgical instrument can be configured as a single sterile use cystoscope, as a flexible endoscope or similar.

According to some further embodiments of the invention, the step of inserting comprises receiving an end of the ac-cessory port in a working channel and positioning a projec-tion element in relation to the housing, wherein the projec-tion element projects from a shell surface of the accessory port and is configured to provide the form fit in interac-tion with the housing.

Optionally, the shaft can be configured as a round tube. The working channel can be configured as a round tube. The working channel can be arranged concentrically in the shaft.

The shaft configured as a round tube can have a substantially circular cross-section with a substantially constant diameter along the axial direction. The working channel configured as a round tube can have a substantially circular cross-section with a substantially constant diameter along the axial direction.

A bearing element of the surgical instrument can grip the shaft or the working channel in such a way that axial displacement and, if necessary, rotation of the working channel relative to the shaft is possible.

Alternatively, a rotation of the working channel relative to the shaft can be blocked or limited, for example, by a longitudinal groove or a flattened area of the working channel. In this way, unintentional rotation of the working channel relative to the shaft can also be avoided in relation to the surgical instrument, thus ensuring perfect functioning of the surgical instrument.

The operating interface and the surgical instrument configured in this way are advantageously particularly easy to manufacture.

### BRIEF DESCRIPTION OF THE DRAWINGS

The present invention is explained more specifically below on the basis of the exemplary embodiments indicated in the schematic figures, in which:
Figs. 1A, 1B show a schematic illustration of a handheld surgical instrument comprising an operating interface according to an embodiment of the invention;
Fig. 2 shows a schematic illustration of an enlarged detail view of an operating interface according to an embodiment of the invention;
Fig. 3 shows a schematic cut view of the operating interface according to Fig. 2;
Figs. 4A, 4B show a schematic illustration of a handheld surgical instrument comprising an operating interface according to a further embodiment of the invention; and
Fig. 5 shows a schematic flow chart of a method for manufacturing an operating interface for a surgical instrument according to a further embodiment of the invention.

The accompanying figures are intended to convey a further understanding of the embodiments of the invention. They illustrate embodiments and are used in conjunction with the description to explain principles and concepts of the invention. Other embodiments and many of the cited advantages emerge in light of the drawings. The elements of the drawings are not necessarily shown to scale in relation to one another. Direction-indicating terminology such as for example "at the top", "at the bottom", "on the left", "on the right", "above", "below", "horizontally", "vertically", "at the front", "at the rear" and similar statements are merely used for explanatory purposes and do not serve to restrict the generality to specific configurations as shown in the figures.

In the figures of the drawing, elements, features and components that are the same, have the same function and have the same effect are each provided with the same reference signs - unless explained otherwise.

### DETAILED DESCRIPTION OF DRAWINGS

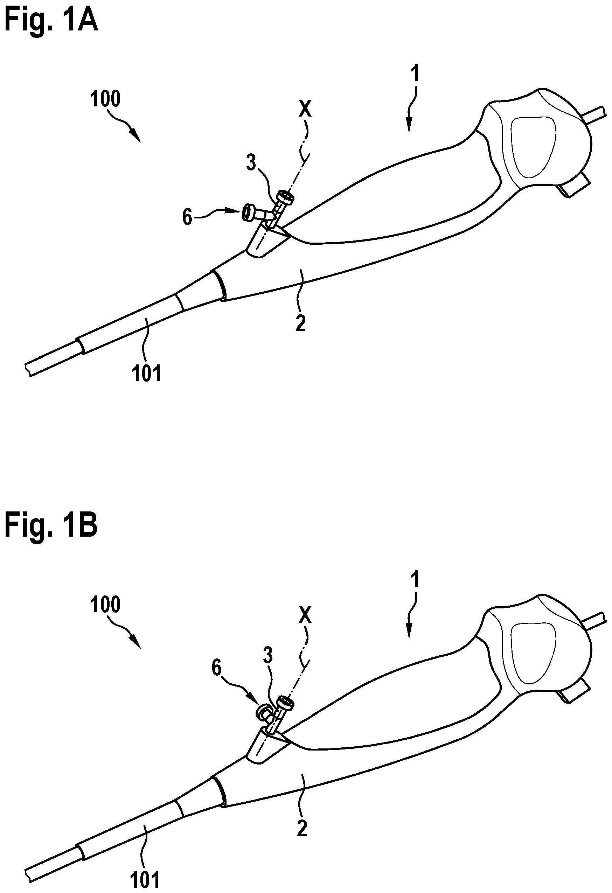

Fig. 1A and 1B show a schematic illustration of a handheld surgical instrument 100 comprising an operating interface 1.

The handheld surgical instrument 100 is configured as an endoscope. The endoscope 100 comprises an operating interface 1 and a shaft 101, which encloses a working channel 102, wherein the shaft 101 is coupled to the operating interface 1. The shaft 101 and the working channel 102 are configured as a round tube. Furthermore, the shaft 101 is arranged concentric in the operating interface 1.

The operating interface 1 is exemplarily configured as an endoscope handle for manual operation by a user. Alternatively, the operating interface can be configured as a corresponding interface for a robot for guidance by a robot, not shown. The operating interface 1 comprises a housing 2 and an accessory port 3.

The housing 2 includes a first part 2a and a second part 2b, which is mountable to the first part 2a.

The accessory port 3 is configured for transmitting fluids between an inside and an outside of the housing 2. Moreover, the accessory port 3 is integrated in the housing 2 and pivotable about a rotation axis X in relation to the housing 2.

In Fig. 1A and 1B, the accessory port 3 comprises a luer connector 6, which is arranged outside the housing 2. The accessory port 3 is pivotable about the rotation axis X such that an orientation of the luer connector 6 changes in relation to an orientation of the housing 2. Specifically, Fig. 1A shows the luer connector 6 in a central orientation. In difference to that, Fig. 1B shows the luer connector 6 in an orientation with a rotation angle of about 90 ° in relation to the central orientation. It is emphasized, that the orientation with a rotation angle of about 90 ° in relation to the central orientation can be provided at both lateral sides of the housing 2. Hence, the luer connector 6 can be pivoted on either side of the endoscope handle, for example to be used by an assistant for irrigation. Therefore, the user can operate the endoscope handle 1 with his left or his right hand and pivot the accessory port 3 such that the user is not disturbed by an accessory connected to the luer connector 6. The user can be a doctor, in particular a surgeon.

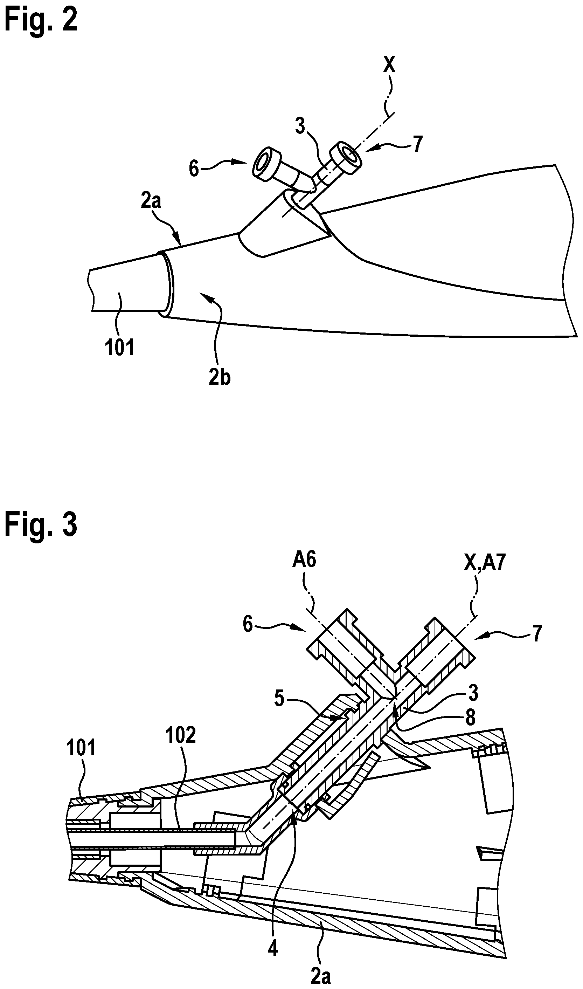

Fig. 2 shows a schematic illustration of an enlarged detail view of an operating interface 1. Fig. 3 shows a schematic cut view of the operating interface 1 according to Fig. 2

The operating interface can substantially comprise the features of the operating interface according to Figs. 1A and 1B. Here, the accessory port 3 can be fluidly connected to the working channel 102, which is arranged inside the housing 2, as it is illustrated in Fig. 3.

Furthermore, the accessory port 3 is integrated in the housing 2 by a form fit. Thereby, the accessory port 3 is enclosed circumferentially by the housing 2. The form fit can be provided by the first part 2a and the second part 2b of the housing. The first part 2a and the second part 2b can be mounted together, in particular mounted by a fastening device, by a bonded connection or similar.

As it is specifically illustrated in Fig. 3, the accessory port 3 comprises an end 4, which is arranged inside the housing 2 and received in the working channel 102. Further, the accessory port 3 comprises a projection element 5, which projects from a shell surface of the accessory port 3 and is configured to provide the form fit in interaction with the housing 2.

The accessory port 3 comprises a first luer connector 7 having a first longitudinal axis A7 and a second luer connector 6 having a second longitudinal axis A6. The first longitudinal axis A7 substantially corresponds to the rotation axis X of the accessory port 3. The second longitudinal axis A6 is substantially perpendicular in relation to the first longitudinal axis A7. Here, the accessory port 3 has a T-shape, but the invention is not limited to such a shape and can also comprise each angle, in particular an angle from 65 ° to 115 °, between the first longitudinal axis A7 and the second longitudinal axis A6.

An intersection 8 of the first longitudinal axis 7 and the second longitudinal axis 6 is arranged outside the housing 2 in this example.

Optionally, the accessory port 3 can comprise means, which are configured to support an orientation of the accessory port 3 at a predefined rotation angle. The predefined rotation angle could be a central orientation of the accessory port 3 or the second luer connector 6, respectively. Alternatively or additionally, the predefined rotation angle could be an orientation with a rotation angle of about 90 ° in relation to the central orientation. In particular, the means are configured such that the accessory port 3 can click in to place in the predefined rotation angle.

Here, the first luer connector 7 or the first longitudinal axis A7, respectively, comprises an angle of about 45 ° or 135 ° in relation to a longitudinal axis of the working channel 102. Furthermore, the rotation axis X comprises an angle of about 45 ° or 135 ° in relation to the longitudinal axis of the working channel 102.

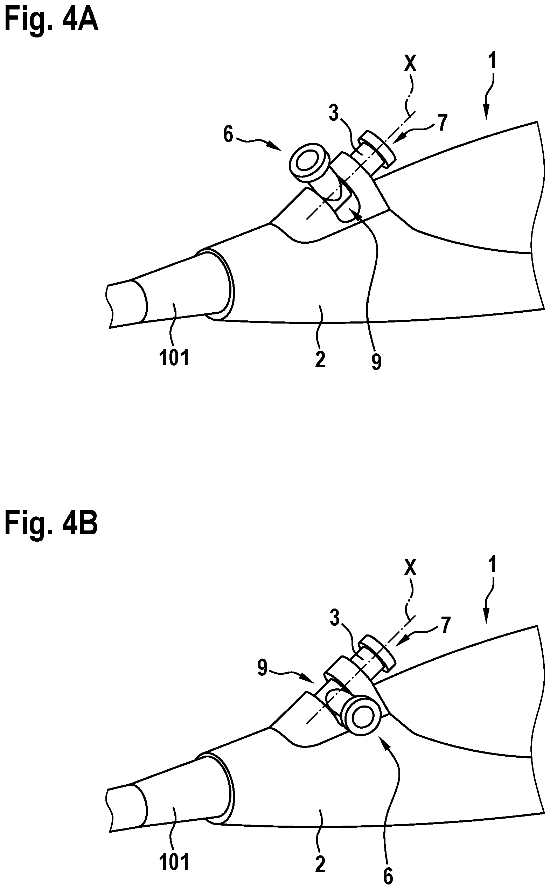

Figs. 4A and 4B show a schematic illustration of a handheld surgical instrument 100 comprising an operating interface 1.

The handheld surgical instrument 100 is configured as an endoscope. The endoscope 100 comprises an operating interface 1 and a shaft 101, which encloses a working channel 102, wherein the shaft 101 is coupled to the operating interface 1. The shaft 101 and the working channel 102 are configured as a round tube. Furthermore, the shaft 101 is arranged concentric in the operating interface 1.

The operating interface 1 is exemplarily configured as an endoscope handle for manual operation by a user. Alternatively, the operating interface can be configured as a corresponding interface for a robot for guidance by a robot, not shown. The operating interface 1 comprises a housing 2 and an accessory port 3.

The housing 2 includes a first part 2a and a second part 2b, which is mountable to the first part 2a.

The accessory port 3 is configured for transmitting fluids between an inside and an outside of the housing 2. Moreover, the accessory port 3 is integrated in the housing 2 and pivotable about a rotation axis X in relation to the housing 2.

In Fig. 4A and 4B, the accessory port 3 comprises a luer connector 6, which is arranged outside the housing 2. The accessory port 3 is pivotable about the rotation axis X such that an orientation of the luer connector 6 changes in relation to an orientation of the housing 2. Specifically, Fig. 4A shows the luer connector 6 in a central orientation. In difference to that, Fig. 4B shows the luer connector 6 in an orientation with a rotation angle of about 90 ° in relation to the central orientation. It is emphasized, that the orientation with a rotation angle of about 90 ° in relation to the central orientation can be provided at both lateral sides of the housing 2. Hence, the luer connector 6 can be pivoted on either side of the endoscope handle, for example to be used by an assistant for irrigation. Therefore, the user can operate the endoscope handle 1 with his left or his right hand and pivot the accessory port 3 such that the user is not disturbed by an accessory connected to the luer connector 6. The user can be a doctor, in particular a surgeon.

Here, an intersection 8 of the first longitudinal axis A7 and the second longitudinal axis A6 is arranged inside the housing 2 such that the second luer connector 6 protrudes through a recess 9 of the housing 2. That means that the first luer connector and the second luer connector would protrude through different recesses of the housing. The orientation of the second luer connector 6 can be limited by an end of the recess 9. In particular, the recess comprises an end on each lateral side of the housing 2 such that the second luer connector 6 can be rotated to each lateral side about 90° starting from the central orientation.

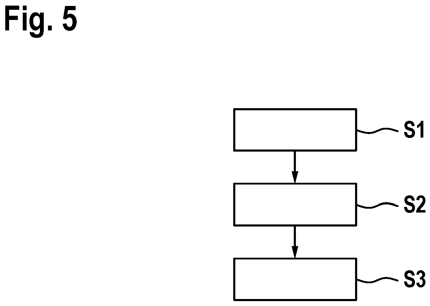

Fig. 5 shows a schematic flow chart of a method for manufacturing an operating interface 1 for a surgical instrument 100. In particular, the method is for manufacturing an operating interface 1 according to Figs. 1A to 3.

The method comprises the steps of providing S1, inserting S2 and mounting S3.

In the step of providing S1, a first part 2a of a housing 2 is provided. The first part 2a could be a half of the housing 2, wherein the housing would be divided lengthwise.

In the step of inserting S2, an accessory port 3 for transmitting fluids between an inside and an outside of the housing 2 is inserted into the first part 2a of the housing 2. Furthermore, the step of inserting comprises receiving an end 4 of the accessory port 3 in a working channel 102. Thereby, a projection element 5 can be positioned in relation to the housing 2, wherein the projection element 5 projects from a shell surface of the accessory port 3 and is configured to provide the form fit in interaction with the housing 2.

In the step of mounting S3, a second part 2b of the housing 2 is mounted to the first part 2a of the housing, wherein the accessory port 3 is integrated in the housing 2 and pivotable about a rotation axis X in relation to the housing 2.

In the detailed description above, various features have been combined in one or more examples in order to improve the rigorousness of the illustration. However, it should be clear in this case that the above description is of merely illustrative but in no way restrictive nature. It serves to cover all alternatives, modifications and equivalents of the various features and exemplary embodiments. Many other examples will be immediately and directly clear to a person skilled in the art on the basis of his knowledge in the art in consideration of the above description.

The exemplary embodiments have been chosen and described in order to be able to present the principles underlying the invention and their application possibilities in practice in the best possible way. As a result, those skilled in the art can optimally modify and utilize the invention and its various exemplary embodiments with regard to the intended purpose of use. In the claims and the description, the terms "including" and "having" are used as neutral linguistic concepts for the corresponding terms "comprising". Furthermore, use of the terms "a", "an" and "one" shall not in principle exclude the plurality of features and components described in this way.

While at least one exemplary embodiment of the present invention(s) is disclosed herein, it should be understood that modifications, substitutions and alternatives may be apparent to one of ordinary skill in the art and can be made without departing from the scope of this disclosure. This disclosure is intended to cover any adaptations or variations of the exemplary embodiment(s). In addition, in this disclosure, the terms "comprise" or "comprising" do not exclude other elements or steps, the terms "a" or "one" do not exclude a plural number, and the term "or" means either or both. Furthermore, characteristics or steps which have been described may also be used in combination with other characteristics or steps and in any order unless the disclosure or context suggests otherwise. This disclosure hereby incorporates by reference the complete disclosure of any patent or application from which it claims benefit or priority.

### REFERENCE LIST

- 1: operating interface
- 2: housing
- 2a: first part of the housing
- 2b: second part of the housing
- 3: accessory port
- 4: end of the accessory port
- 5: projecting element
- 6: second luer connector
- 7: first luer connector
- 8: intersection
- 9: recess

- 100: surgical instrument
- 101: shaft
- 102: working channel

- A6: second longitudinal axis
- A7: first longitudinal axis
- X: rotation axis

- S1: providing
- S2: inserting
- S3: mounting

## Claims

1. Operating interface (1) for a surgical instrument (100), in particular for an endoscope, comprising:
a housing (2) having a first part (2a) and a second part (2b), which is mountable to the first part (2a); and
an accessory port (3) for transmitting fluids between an inside and an outside of the housing (2), wherein the accessory port (3) is integrated in the housing (2) and pivotable about a rotation axis (X) in relation to the housing (2).

2. Operating interface (1) according to claim 1,
**characterized in that**
the accessory port (3) is integrated in the housing (2) by a form fit.

3. Operating interface (1) according to claim 1 or 2,
**characterized in that**
the accessory port (3) comprises an end (4), which is arranged inside the housing (2) and received in a working channel (102), and a projection element (5), which projects from a shell surface of the accessory port (3) and is configured to provide the form fit in interaction with the housing (2).

4. Operating interface (1) according to one of the preceding claims,
**characterized in that**
the accessory port (3) comprises at least one luer connector (6), which is arranged outside the housing (2), wherein the accessory port (3) is pivotable about the rotation axis (X) such that an orientation of the at least one luer connector (6) changes in relation to an orientation of the housing (2).

5. Operating interface (1) according to one of the preceding claims,
**characterized in that**
the accessory port (3) comprises a first luer connector (7) having a first longitudinal axis (A7) and a second luer connector (6) having a second longitudinal axis (A6), wherein the first longitudinal axis (A7) substantially corresponds to the rotation axis (X) of the accessory port (3).

6. Operating interface (1) according to claim 5,
**characterized in that**
the second longitudinal axis (A6) is substantially perpendicular in relation to the first longitudinal axis (A7).

7. Operating interface (1) according to claim 5 or 6,
**characterized in that**
an intersection (8) of the first longitudinal axis (A7) and the second longitudinal axis (A6) is arranged inside the housing (2) such that the second luer connector (6) protrudes through a recess (9) of the housing (2).

8. Operating interface (1) according to claim 5 or 6,
**characterized in that**
an intersection (8) of the first longitudinal axis (7) and the second longitudinal axis (6) is arranged outside the housing (2).

9. Operating interface (1) according to one of the preceding claims,
**characterized in that**
the accessory port (3) comprises means, which are configured to support an orientation of the accessory port (3) at a predefined rotation angle.

10. Operating interface (1) according to one of the preceding claims,
**characterized in that**
the operating interface (1) is configured as an endoscope handle for manual operation by a user.

11. Surgical instrument (100), in particular endoscope, comprising:
an operating interface (1) according to one of the preceding claims; and
a shaft (101), which encloses a working channel (102), wherein the shaft (101) is coupled to the operating interface (1).

12. Surgical instrument (100) according to claim 11,
**characterized in that**
the shaft (101) and the working channel (102) are configured as a round tube, and wherein the shaft (101) is arranged concentric in the operating interface (1).

13. Method for manufacturing an operating interface for a surgical instrument, in particular for manufacturing an operating interface (1) according to one of the claims 1 to 10, comprising the steps of:
providing (S1) a first part (2a) of a housing (2);
inserting (S2) an accessory port (3) for transmitting fluids between an inside and an outside of the housing into the first part (2a) of the housing; and
mounting (S3) a second part (2b) of the housing (2) to the first part (2a) of the housing, wherein the accessory port (3) is integrated in the housing (2) and pivotable about a rotation axis (X) in relation to the housing.

14. Method according to claim 13,
**characterized in that**
the step of inserting (S2) comprises receiving an end (4) of the accessory port (3) in a working channel (102), and positioning a projection element (5) in relation to the housing (2), wherein the projection element (5) projects from a shell surface of the accessory port (3) and is configured to provide the form fit in interaction with the housing (2).
